(19) Europäisches Patentamt
European Patent Office
Office européen des brevets

(11) Veröffentlichungsnummer : **0 149 174**
**B1**

(12) # EUROPÄISCHE PATENTSCHRIFT

(45) Veröffentlichungstag der Patentschrift : **23.03.88**

(21) Anmeldenummer : **84115673.0**

(22) Anmeldetag : **18.12.84**

(51) Int. Cl.$^4$ : **C 07 C101/24**, C 07 C 99/00, A 61 K 7/40, A 61 L 2/16, C 11 D 3/48, C 11 D 9/50

(54) **Neue amphotere Verbindungen, diese Verbindungen enthaltende Gemische zur Desinfektionsreinigung sowie Verfahren zur Herstellung dieser Verbindungen und Gemische.**

(30) Priorität : **30.12.83 DE 3347534**

(43) Veröffentlichungstag der Anmeldung : **24.07.85 Patentblatt 85/30**

(45) Bekanntmachung des Hinweises auf die Patenterteilung : **23.03.88 Patentblatt 88/12**

(84) Benannte Vertragsstaaten : **BE CH DE FR GB LI NL SE**

(56) Entgegenhaltungen :
**DE-B- 1 241 457**
**DE-C- 845 941**
**DE-C- 947 972**
**US-A- 2 840 600**

(73) Patentinhaber : **HOECHST AKTIENGESELLSCHAFT**
**Postfach 80 03 20**
**D-6230 Frankfurt am Main 80 (DE)**

(72) Erfinder : **Blaschke, Günter, Dr.**
**Bajuwarenstrasse 36**
**D-8261 Winhöring (DE)**
Erfinder : **May, Adolf, Dr.**
**Dahlienweg 5**
**D-6238 Hofheim am Taunus (DE)**
Erfinder : **Bücking, Hans-Walter, Dr.**
**In den Padenwiesen 30**
**D-6233 Kelkheim (Taunus) (DE)**
Erfinder : **Wallhäusser, Karl H., Prof. Dr.**
**Lessingstrasse 20**
**D-6238 Hofheim am Taunus (DE)**

**EP 0 149 174 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Amphotere Verbindungen des Typs Alkylpoly- [ethylenamino]-glycine mit der allgemeinen Formel $R—NH(C_2H_4NH)_nCH_2—COOH$ (R = längerkettiger Alkylrest, n = 1 oder mehrfaches von 1) sind bekannte Stoffe mit mikrobizider und algizider Wirkung, wie sie beispielsweise für die Desinfektion der Hände, für die Desinfektion harter Oberflächen und für den Infektionsschutz in der Lebensmittel- und Getränkeindustrie Verwendung finden. Verbindungen des genannten Typs werden aus Alkylpoly- [ethylenamin]-Verbindungen hergestellt, die ihrerseits üblicherweise durch Umsetzung von Alkylhalogeniden mit überschüssigem Ethylendiamin, Diethylentriamin oder Triethylentetraamin gewonnen werden. Die so entstandenen Verbindungen vom Typ Alkylpoly- [ethylenamin] werden anschließend mit ω-Halogencarbonsäuren oder endständig ungesättigten Alkencarbonsäuren oder deren Salzen umgesetzt. Die Herstellung von Verbindungen dieses Typs ist beispielsweise beschrieben in der DE-PS 812 105, DE-PS 947 972 oder DE-PS 856 042. Die Herstellung dieser an sich als Mikrobizide gut wirksamen amphoteren Verbindungen ist jedoch mit erheblichen Nachteilen behaftet. Um monosubstituierte Verbindungen vom Typ Alkylpoly-[ethylenamin] zu erhalten (das heißt unter Vermeidung der weiteren Addition von Alkylhalogenid an die noch vorhandenen primären und sekundären Aminogruppen des Polyalkylenamins), müssen die Alkylhalogenide mit einem 4- bis 5-fachen molaren Überschuß an den obengenannten Polyaminen umgesetzt werden. Nach dieser Umsetzung muß das überschüssige Amin abdestilliert werden. Trotz des hohen Überschusses an Polyalkylenamin entstehen dennoch als Nebenprodukte di-, tri- und tetraalkylsubstituierte Derivate dieser Polyalkylenamine. Diese in erheblichen Mengen anwesenden Nebenprodukte ergeben nach der Überführung in die amphotere Verbindung mittels der genannten Halogencarbonsäuren oder endständig ungesättigten Alkencarbonsäuren Trübungen und Ausfällungen, wirken also qualitätsmindernd. Ebenso führt die genannte destillative Entfernung der Polyalkylenamine zu dunkel gefärbten, qualitativ ebenfalls wenig befriedigenden Produkten. Deshalb muß im allgemeinen das gewünschte Alkylpolyaminoethylen vor der Überführung in die amphotere Verbindung ebenfalls destilliert werden.

Um diese aufwendige Darstellung und Reinigung der genannten amphoteren Verbindungen zu umgehen, ist auch schon beschrieben worden (siehe beispielsweise DE-PS 1 041 627) Alkylpoly-[trimethylenamino]-glycine der Formel $R—NH(CH_2CH_2CH_2NH)_nCH_2COOH$ als Mikrobizide einzusetzen. Die Vorstufen dieser Klasse von amphoteren Verbindungen, nämlich Polyamine vom Typ Alkylpoly-[trimethylenamin] werden durch Addition von Acrylnitril an primäre Alkylamine und anschließende katalytische Hydrierung der so entstandenen Alkylaminopropionitrile mit Hilfe von Raney-Nickel und Wasserstoff hergestellt. Diese Addition von Acrylnitril kann mehrmals wiederholt werden. Die Umsetzung der schließlich erhaltenen Hydrierungsprodukte mit ω-Halogencarbonsäuren oder endständig ungesättigten Carbonsäuren in der oben beschriebenen Weise führt dann zu dem vorgenannten Typ von Alkylpoly-[trimethylenamino]-glycinen, deren mikrobizide Wirkung derjenigen der Alkylpoly-[ethylenamino]-glycine etwa entspricht. Nachteilig wirkt sich jedoch die jeweils um eine $CH_2$-Einheit verlängerte Polyaminoalkylenkette auf die Löslichkeit dieser amphoteren Verbindungen aus. Diese schlechtere Löslichkeit kann in wäßrigen Einstellungen zu Ausfällungen führen. Auch ist die mehrstufige Synthese (wegen der mehrstufigen Anlagerung von Acrylnitril) relativ aufwendig.

In der DE-C-845 941 wird die Herstellung von Aminosäuren mit keimtötender Wirkung beschrieben, wobei ein α,ω-Dialkylpolyalkylenpolyamin mit einer Halogencarbonsäure zur angestrebten Aminosäure umgesetzt wird, worauf gegebenenfalls aus der Aminosäure mit einer anorganischen oder organischen Säure ein wasserlösliches Salz gebildet wird.

Aus der DE-B-1 241 457 ist ein Verfahren zur Herstellung von N-Aminopropyl-aminocarbonsäuren der Formel $NH_2(CH_2)_3NHC(R,R')COOH$ bekannt, bei dem man N-Cyanethylaminocarbonsäure der Formel $NC(CH_2)_2NHC(R,R')COOH$ bei einer Temperatur von 60 bis 120 °C mit Hilfe von Raney-Nickel in Essigsäureanhydrid katalytisch hydriert (R und R' = H, eine Alkyl-, Aryl- oder eine Aralkylgruppe).

Schließlich sind aus der US-A-2 840 600 Verbindungen der Formel $RNH(CH_2)_3NHCH(CH_3)CH_2COOH$ (R = Alkyl) bekannt. Sie werden durch Umsetzung von beispielsweise N-substituiertem Trimethylendiamin mit einer niederen ungesättigten Carbonsäure, beispielsweise Methacrylsäure, erhalten. Bezüglich der Eigenschaften dieser Verbindungen gilt im wesentlichen das, was oben über die bekannten Alkylpoly-[trimethylenamino]-glycine gesagt worden ist.

Es besteht daher ein Bedürfnis nach Verbindungen mit desinfizierender Wirkung, die die Vorteile dieser Klasse von amphoteren Verbindungen besitzen, dennoch aber die obengenannten Nachteile nicht aufweisen.

Gemäß der vorliegenden Erfindung wird diesem Bedürfnis Rechnung getragen durch amphotere Verbindungen der Formel

$$R^1-N\begin{cases}(CH_2)_a-NH-(CH_2)_c-COOH \\ (CH_2)_b-NH_2\end{cases} \cdot x\ HA \qquad (I)$$

worin

$R^1$ ein Alkyl- oder Alkenylrest mit 8 bis 22 C-Atomen ist ;

A das Anion einer Mineral- oder Carbonsäure ist ;

a und b — unabhängig voneinander — Werte von 2 oder 3 ;

c Werte von 1 oder 2 annehmen kann ; und

x 0 ist oder ganzzahlige Werte von 1 bis 3 annehmen kann.

Vorzugsweise ist in diesen Verbindungen der Formel I $R^1$ ein Alkylrest mit 8 bis 14 C-Atomen und a und b haben den Wert 3. A ist vorzugsweise Cl, Br, Hydrogenphosphat, Actetat, Lactat oder Glykolat.

Diese erfindungsgemäßen amphoteren Verbindungen der Formel I sind sehr gut in Wasser löslich, und sie zeigen gegenüber den obengenannten bekannten amphoteren Verbindungen sogar leicht verbesserte mikrobizide Eigenschaften. Sie sind auf dem im folgenden beschriebenen Weg leicht und ohne Bildung von Nebenprodukten zugänglich. Reinigungsoperationen des gewonnenen Endproduktes der Formel I oder von Zwischenstufen sind nicht erforderlich.

In diesen erfindungsgemäßen Verbindungen der Formel I besitzt der Rest $R^1$ 8 bis 22 C-Atome, er kann gesättigt oder ungesättigt mit 1 bis 3 olefinischen Doppelbindungen, und er kann geradkettig oder verzweigt sein. Diese Alkyl- oder Alkenylreste, die dem primären Ausgangsamin bei der Herstellung der erfindungsgemäßen Verbindungen der Formel I entstammen, sind häufig Gemische oder Kettenschnitte, bevorzugt mit der Kettenverteilung der Reste von natürlichen Fettsäuren, wie insbesondere der Cocos-, Talg- oder Palmkernfettsäure, aus denen diese Ausgangsamine über den Weg der Nitrilhydrierung oder der Ammonolyse der entsprechenden Fettalkohole gewonnen werden. Die zur Herstellung der primären Amine mittels Ammonolyse verwendeten Alkohole können neben Fettalkoholen auch solche mit gerader oder verzweiger Kette aus dem Ziegler-Prozeß (Ethylen-Aufbaualkohole) oder aus der Oxosynthese sein.

Zur Herstellung der erfindungsgemäßen Verbindungen der Formel I wird zunächst ein solches primäres Amin der Formel $R^1NH_2$ (IV), worin $R^1$ die obengenannte Bedeutung hat, mit 2 mol mindestens eines reaktionsfähigen Nitrils mit 2 bis 3 C-Atomen (einschließlich der CN-Gruppe) zu einer Verbindung der allgemeinen Formel

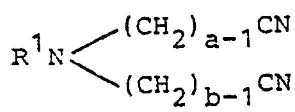

(V)

worin $R^1$, a und b die obengenannte Bedeutung haben, in einer Dicyanalkylierungsreaktion umgesetzt. Diese Reaktion ist bekannt, beispielsweise aus der US-PS 3 028 415. Sie kann sowohl mit saurer als auch mit basischer Katalyse, mit Hilfe von Lösungsmitteln, wie Wasser oder auch niederkettigen Alkoholen, drucklos oder unter erhöhtem Druck, kontinuierlich oder diskontinuierlich durchgeführt werden. Als saure Katalysatoren werden Essigsäure, Phosphorsäure, Salzsäure und andere Mineralsäuren genannt (US-PS 3 615 797, US-PS 3 028 415, DE-OS 1 941 913), als basische Katalysatoren sind empfohlen worden Natrium- oder Kaliumhydroxid, Alkalialkoholate, Trimethylbenzylammoniumhydroxid und Morpholin (Kirk-Othmer, Encyclopedia of Chemical Technology, 1965, Band 6, Seite 634 ff. ; H. A. Bruson « Cyanoethylation », Organic Reactions 5, 1949, Seite 79 ff., Verlag John Wiley and Sons, New York). Als Co-Katalysatoren oder auch als Lösungsvermittler werden Wasser oder niedere Alkohole, wie Methanol, Ethanol, Isopropanol oder Gemische derselben, in Anteilen von 1 bis 20 Gew.-% zugegeben. Die Dicyanalkylierung wird unter Normaldruck oder leichtem bis mittlerem Überdruck von 1 bis 20 bar, gegebenenfalls in Gegenwart eines Inertgases, und bei Temperaturen von 60 bis 150 °C durchgeführt. Das Cyanalkylierungsmittel, vorzugsweise Acrylnitril, Chloracetonitril oder ω-Chlorpropionitril, wird stöchiometrisch oder in einem bis zu vierfachen Überschuß angewandt.

Anschließend wird das so gewonnene Dicyanalkylierungsprodukt (V) in Gegenwart von Wasserstoff zu einer Verbindung der Formel

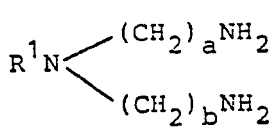

(VI)

worin wiederum $R^1$, a und b die obengenannte Bedeutung haben, reduziert.

Das bei dieser Reduktion erhaltene Amin der Formel VI wird in Wasser dispergiert und mit einer ω-Halogencarbonsäure der Formel X $(CH_2)_cCOOH$ (VII), worin X = Halogen, vorzugsweise Cl oder Br ist und c die in Formel I genannte Bedeutung hat, wie beispielsweise mit ω-Halogenpropionsäure, Halogenessigsäure, oder mit Acrylsäure oder auch mit den Alkali- oder Erdalkalisalzen, den Estern oder den Nitrilen, die sich von den genannten Carbonsäuren ableiten, umgesetzt und gegebenenfalls — im Falle der Ester und Nitrile — zu den freien Carbonsäuren hydrolysiert und gegebenenfalls mit Mineral- oder Carbonsäuren unter Bildung der Salze neutralisiert wird. Bei der Umsetzung wird ein Molverhältnis von Amin der Formel VI : Carbonsäure oder Carbonsäurederivat von 1 : 1 eingehalten. Die Umsetzung

läuft ab bei Temperaturen von 80 bis 100 °C. Wird die Umsetzung mit den genannten Halogencarbonsäuren durchgeführt, so fällt die amphotere Verbindung als Hydrohalogenid an, woraus die freie amphotere Verbindung der Formel I ($x = 0$) durch Behandlung mit Alkalien in Freiheit gesetzt werden kann.

Die so hergestellten, erfindungsgemäßen amphoteren Verbindungen der Formel I sind Mikrobizide mit sehr guten desinfizierenden Eigenschaften, wie aus Tabelle 2 ersichtlich ist, und sie sind in wäßrigen Einstellungen mit einer Wirkstoffkonzentration von bis zu 50 Gew.-%, in alkoholischen Einstellungen mit einer Wirkstoffkonzentration von bis zu 90 Gew.-% gewinnbar, da sie bei der Überführung des Polyamins in die amphotere Verbindung keine sogenannte Gelphase (das heißt, den Zustand einer nicht mehr rührbaren, hochviskosen Mischung) durchlaufen. Diese sonst auftretende Gelphase verhindert bei den oben besprochenen Verbindungen des Standes der Technik wäßrige Einstellungen von mehr als 30 Gew.-% an Wirkstoff.

Überraschenderweise hat es sich auch gezeigt, daß die erfindungsgemäßen Verbindungen der Formel I ebenfalls den obengenannten bekannten amphoteren Verbindungen vom Typ Alkylpoly-[trimethylenamin] — wie sie unten in Formel II definiert werden — eine verbesserte Wasserlöslichkeit zu erteilen vermögen und daher diese zum Einsatz in Desinfektionsreiniger-Zusammensetzungen geeigneter machen. Solche Gemische können — sei es entweder von der Methode der Herstellung her oder sei es als hinzugefügte Mischungskomponenten — gegebenenfalls noch einen Anteil an amphoteren Verbindungen der unten definierten Formel III enthalten.

Gemäß dieser Erfindung werden somit auch Desinfektionsreiniger-Zusammensetzungen zur Verfügung gestellt, die dadurch gekennzeichnet sind, daß sie

a) 80 bis 30 Mol-% einer amphoteren Verbindung der Formel I gemäß Anspruch 1, und

b) 20 bis 70 Mol-% einer amphoteren Verbindung der Formel

$$R^2—NH—(CH_2)_d—NH—(CH_2)_e—COOH \cdot y\ HA \qquad (II),$$

worin

$R^2$ eine der in Formel I für $R^1$ genannten Bedeutungen hat ;

A das Anion einer Mineral- oder Carbonsäure ist ;

$d = 3$ ist ; e Werte von 1 oder 2 und y 0 ist oder Werte von 1 oder 2 annehmen kann,

enthalten, wobei 0 bis 50 % der Molmenge von Komponente a) durch

c) eine amphotere Verbindung der Formel

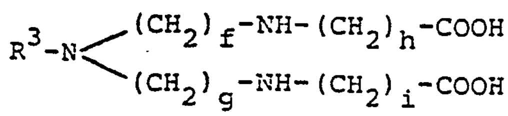 · z HA (III)

worin

$R^3$ eine in der in Formel I für $R^1$ genannten Bedeutungen hat ;

A das Anion einer Mineral- oder Carbonsäure ist ;

f und g — unabhängig voneinander — Werte von 2 oder 3 haben ;

h und i — unabhängig voneinander — Werte von 1 oder 2 haben ; und

z 0 ist oder ganzzahlige Werte von 1 bis 3 annehmen kann,

ersetzt sein kann.

Vorzugsweise können 0 bis 25 % der Molmenge der Komponente a) durch die Komponente c) ersetzt sein.

Wie oben bereits aufgezeigt, handelt es sich bei den Verbindungen der Formel II, die die Komponente b) bilden, um bekannte Verbindungen, die nach bekannten Verfahren hergestellt werden können. Desgleichen sind die Verbindungen der Formel III, die gegebenenfalls als Komponente c) im Gemisch enthalten sein können, bekannt aus der JP-AS Sho-40-13846 (vgl. Chem. Abstracts 63, 1965, 17 982 g). Somit kann man zu den obengenannten, als Desinfektionsreiniger dienenden Gemischen gelangen, indem die erfindungsgemäßen Verbindungen der Formel I mit den getrennt hergestellten Verbindungen der Formel I mit den getrennt hergestellten Verbindungen der Formel II und gegebenenfalls der Formel III in einem entsprechenden Verhältnis abgemischt werden. In diesem Fall kann jede im Rahmen der obigen Definition der Substituenten der Formel I liegende Verbindung eingesetzt werden.

Solche Gemische können aber auch erzeugt werden durch eine Variante des oben beschriebenen Herstellungsverfahrens für die erfindungsgemäßen Verbindungen der Formel I. In einem solchen Fall werden die Ausgangsamine $R^1NH_2$ der Formel IV mit einem reaktionsfähigen Nitril mit 3 C-Atomen (einschließlich der CN-Gruppe) umgesetzt, also vorzugsweise mit Acrylnitril oder mit ω-Chlorpropionitril, und zwar in einer Menge von weniger als 2 und von mehr als 1 mol pro 1 mol des genannten Ausgangsamins der Formel IV. Hier läuft also neben der Dicyanalkylierung auch eine Monocyanalkylierung ab, die zu dem genannten Gemisch führt. Vorzugsweise werden 1,8 bis 1,3 mol der genannten Nitrile mit 1 mol des Ausgangsamins umgesetzt.

Das erhaltene Gemisch aus Nitril und Dinitril wird dann in der oben beschriebenen Weise reduziert und in das entsprechende Gemisch amphoterer Verbindungen der Formel I' und II", worin a, b und d = 3

sind, überführt. Vor der Überführung wird der im Gemisch vorhandene Gehalt and tertiärem und an sekundärem Amin-Stickstoff — entsprechend dem Gehalt an den jeweiligen den Formeln I und II analogen Aminen — bestimmt. Wendet man bei der Überführung in die amphotere Verbindung mittels ω-Halogencarbonsäuren, deren obengenannten Derivaten oder mittels Acrylsäure pro 1 Mol-Äquivalent an sekundärem und pro 1 Mol-Äquivalent an tertiärem Amin-Stickstoff je 1 Mol-Äquivalent an den vorgenannten Säuren oder Säurederivaten an, so erhält man das obengenannte Gemisch der Verbindungen der Formel I' und II''. Wendet man pro 1 Mol-Äquivalent an sekundärem Amin-Stickstoff 1 Mol-Äquivalent der genannten Säuren an, steigert jedoch den Anteil dieser Säuren pro 1 Mol-Äquivalent an tertiärem Amin-Stickstoff auf mehr als 1 Mol-Äquivalent, wobei dieser Anteil bis 1,5, vorzugsweise bis 1,25 Mol-Äquivalent betragen kann, so werden in den genannten Gemischen die Verbindungen der Formel I' teilweise, das heißt bis zu 50 %, vorzugsweise bis zu 25 %, ihrer Molmenge durch Verbindungen der Formel III', in der h und i = 3 sind, ersetzt.

Solchen Gemischen, die auf die beschriebene Weise synthetisiert wurden, kann man auch Verbindungen der obengenannten Formel I zusetzen, in der die Indices a und b = 2 sind. Ferner können den erfindungsgemäßen Verbindungen der Formel I oder den oben näher beschriebenen Desinfektionsreiniger-Gemischen auch andere bekannte Verbindungen mit desinfizierender und reinigender Wirkung beigegeben werden. Dies sind beispielsweise quaternäre Ammoniumsalze mit 1 oder 2 längeren Fettalkylresten und 1 oder 2 kurzkettigen Alkylresten, von denen einer auch ein Benzylrest sein kann. Ferner sind dies Verbindungen der Formel

$$R-\overset{R}{\underset{R}{N^{+}}}-(CH_2)_q-COO^- ,$$

wobei in dieser Formel einer der Reste R auch $R—CO—NH—(CH_2)_3—$ sein kann, während die verbleibenden oder alle Reste R Alkylgruppen sind und q = 1 oder 2 ist.

Die erfindungsgemäßen Verbindungen der Formel I und deren Gemische sind hochwirksame Mikrobizide mit sehr guter bakterizider und algizider Wirkung. Sie besitzen eine ausgezeichnete Wasserlöslichkeit und können in Form hochkonzentrierter Einstellungen in Wasser oder Alkoholen oder deren Gemischen mit bis zu 90 Gew.-% Wirkstoffgehalt gewichtssparend versandt und in den Handel gebracht werden, wobei solche Konzentrate dennoch bei Raumtemperatur flüssig sind. Der Mindestgehalt (vorzugsweise mindestens 10 Gew.-%) solcher Einstellungen ist nicht kritisch, da solche hochkonzentrierten Einstellungen vor der Anwendung problemlos mit Wasser und/oder Alkohol auf den gewünschten Wirkstoffgehalt verdünnbar sind. Sie sind besonders geeignet zur Händedesinfektion und auch zur Desinfektion von Gegenständen mit harten Oberflächen, also beispielsweise ärztlichen und zahnärztlichen Instrumenten.

Die erfindungsgemäßen Verbindungen der Formel I und die genannten Gemische können auch als mikrobizide Zusätze bei der Formulierung von Reinigungsmitteln in Kombination mit üblichen anionischen, nicht-ionischen, kationischen und amphoteren Tensiden dienen.

Dafür geeignete anionische Tenside sind beispielsweise Seifen, Fettalkoholsulfate, Alkylethersulfate, Fettsäurekondensationsprodukte, wie Tauride, Methyltauride, Sarkoside, ferner α-Olefinsulfonate, Hydroxyalkansulfonate, sekundäre Alkansulfonate, Amidethersulfate oder Alkylbenzolsulfonate. Als nichtionische Tenside können beispielsweise Polyglykolmonoalkylether und -monoester, Aminoxide und Ethylenoxid-Propylenoxid-Kondensationsprodukte verwendet werden. Daneben ist auch die Kombination mit anderen amphoteren Tensiden wie Alkyl-Betainen, Alkylamido-Betainen, Imidazolinderivaten oder Sulfobetainen möglich. Schließlich können die erfindungsgemäßen Verbindungen der Formel I auch in Abmischung mit kationischen Tensiden wie Cetyltrimethylammoniumchlorid, Cetyltrimethylammoniumbromid, Cetyldimethylbenzylammoniumchlorid, Didecyldimethylammoniumchlorid, Pentaoxyethylstearylammoniumchlorid, quaternierten Etheraminen oder polymeren quartären Ammoniumverbindungen eingesetzt werden. Bevorzugt sind nicht-ionische Tenside. Weitere Zusätze, die auf sonst übliche Weise in Reinigungsmitteln verwendet werden, können mit den erfindungsgemäßen Verbindungen der Formel I gegebenenfalls kombiniert werden. Dies sind beispielsweise viskositätserhöhende oder -erniedrigende Verbindungen wie Celluloseether, Elektrolyte wie beispielsweise Natriumchlorid oder Ammoniumchlorid, Fettsäurepolyglykolester, Alkanolamide, Magnesium-Aluminium-Silicate, Polyglykole, Glycerin und Ethanol. Bei der Verarbeitung zu pulverförmigen Präparaten können weiterhin üblicherweise benutzte Füll- und Trägersubstanzen wie hochdisperse, amorphe Kieselsäure, Natriumsulfat, Magnesium-Aluminium-Silicat, Stärkederivate und dergleichen verwendet werden.

Ferner sind übliche Zusätze Bleichmittel, Chlorabspalter, Chelatbildner und gegebenenfalls auch Kunststoffdispersionen.

In solchen Reinigungsmitteln sind die erfindungsgemäßen Verbindungen der Formel I oder deren Gemische üblicherweise in einem Anteil von 1 bis 40, vorzugsweise von 10 bis 20 Gew.-%, wobei diese Mengen aber bei speziellen Anwendungszwecken auch über- oder unterschritten werden können, enthalten.

Die folgenden Beispiele sollen die Erfindung näher erläutern :

Herstellung der Ausgangsamine der Formel VI bzw. der Amingemische VI'

Beispiel A

In einem 2-Liter-Vierhalskolben mit Rückfluß, Thermometer, Rührer und Dosiergefäß werden 670 g technisches Laurylamin (Zusammensetzung bezüglich der Reste R : $C_{12}$ 73 Gew.-% ; $C_{14}$ 27 Gew.-% ; 3,5 mol), 68 g Wasser, 34 g Methanol und 14 g konzentrierte Essigsäure auf 60 °C erhitzt. Innerhalb einer Stunde tropft man 373 g (7,03 mol) Acrylnitril zu und rührt weitere 24 h bei 75 °C unter Rückfluß. Anschließend wird mit 13 g NaOH und 120 g Wasser neutralisiert, das Waschwasser separiert und das Produkt in Vakuum von Restwasser und Lösungsmittel befreit. Man erhält 1 000 g des entsprechenden Laurylamino-di-propionitrils mit einer Aminzahl von 33,9 und einem tertiären Amingehalt von 96,9 %. Die Bestimmung der Aminzahl (AZ) und des Gehaltes an tertiärem Stickstoff erfolgt durch Titration mit 0,1 N $HClO_4$ in Eisessig bzw. Essigsäureanhydrid.

Ein 5-Liter-Autoklav wird mit 2 020 g des so gewonnenen Laurylamino-di-propionitrils, 3 g Kobalt-Trägerkontakt (Träger : Kieselgur) und 300 ml flüssigen Ammoniaks beschickt. Die Hydrierung erfolgt bei 150 bis 180 bar $H_2$ und 110 bis 140 °C innerhalb von 3 Stunden. Nach Abfiltrieren des Katalysators erhält man 2 010 g eines Gemisches VI' das nahezu quantitativ aus Bis-(3-aminopropyl)-laurylamin besteht und Laurylaminotrimethylenamin in sehr geringer Menge enthält. Es besitzt folgende Kenndaten :

AZ = 97,4 65,8 % Anteile an primären Aminogruppen
2,1 % Anteile an sekundären Aminogruppen
32,1 % Anteile an tertiären Aminogruppen.

Die Bestimmung der Aminzahl und der Aminverteilung erfolgt durch Titration mit 0,2 N-isopropanolischer HCl in wasserfreiem Medium. Die Aminverteilung wird durch Blokkierung des basischen Aminstickstoffs mit Salicylaldehyd (primäres N) bzw. Phenylisothiocyanat (primäres und sekundäres N) durchgeführt.

Beispiel B

670 g des Laurylamins aus Beispiel A (3,5 mol) werden wie in Beispiel A bereits beschrieben, mit 335 g (6,3 mol) Acrylnitril umgesetzt und dann hydriert. Das Amingemisch VI', bestehend aus 81,8 Gew.-% Bis-(3-aminopropyl)-laurylamin und 18,2 Gew.-% Laurylaminotrimethylenamin, weist folgende Kenndaten auf :

AZ = 95,0 63,3 % Anteile an primären Aminogruppen
9,1 % Anteile an sekundären Aminogruppen
27,6 % Anteile an tertiären Aminogruppen.

Beispiel C

670 g des Laurylamins aus Beispiel A (3,5 mol) werden, wie in Beispiel A beschrieben, mit 248 g (4,7 mol) Acrylnitril umgesetzt und dann hydriert. Das Amingemisch VI', bestehend aus 34,6 Gew.-% Bis-(3-aminopropyl)-laurylamin und 65,4 Gew.-% Laurylaminotrimethylenamin, weist folgende Kenndaten auf :

AZ = 86,4 55,5 % Anteile an primären Aminogruppen
32,7 % Anteile an sekundären Aminogruppen
11,8 % Anteile an tertiären Aminogruppen.

Herstellung der erfindungsgemäßen Verbindungen und Gemische

Beispiel 1

308 g Amin aus Beispiel A) und 829 g Wasser werden in einem 2-Liter-Reaktionsgefäß vorgelegt und unter Rühren auf 90 °C erwärmt. Bei dieser Temperatur werden innerhalb 1 h 99,2 g Chloressigsäure zugegeben. Anschließend läßt man weitere 5 h bei 95 °C nachreagieren. Die Analyse des Produkts erfolgt mittels HPLC.

Beispiel 2

308 g Amin aus Beispiel A) werden in 210 g Isopropanol in einem 2-Liter-Reaktionsgefäß vorgelegt und unter Rühren auf 60 °C erwärmt. Bei dieser Temperatur werden innerhalb 3 h 88,7 g Acrylsäuremethylester zugetropft. Danach wird noch 5 h bei 60 °C nachgerührt. Durch Zugabe von 275 g einer 15 gew.-%igen wäßrigen Natronlauge wird die Additionsverbindung in das entsprechende Natriumsalz überführt. Bei 50 °C und Wasserstrahlvakuum werden 50 g Lösemittel abdestilliert, um von Methanol zu befreien. Mit Wasser wird auf 50 Gew.-% Feststoff eingestellt. Die Analyse des Produkts erfolgt mittels HPLC.

Beispiel 3

295,2 g Amin aus Beispiel B) und 843,2 g Wasser werden in einem 2-Liter-Reaktionsgefäß vorgelegt

und unter Rühren auf 90 °C erwärmt. Bei dieser Temperatur werden innerhalb 1 h 99,2 g Chloressigsäure zugegeben und anschließend weitere 5 h bei 95 °C nachreagiert. Die Analyse des Produkts erfolgt mittels HPLC.

Beispiel 4

295,2 g Amin aus Beispiel B) und 825 g Wasser werden in einem 2-Liter-Reaktionsgefäß vorgelegt und unter Rühren auf 90 °C erwärmt. Bei dieser Temperatur werden innerhalb 1 h 118,1 g Chloressigsäure zugegeben und weitere 5 h bei 95 °C nachreagiert. Die Analyse des Produkts erfolgt mittels HPLC.

Beispiel 5

410,3 g Amin aus Beispiel C) und 1 178 g Wasser werden in einem 2-Liter-Reaktionsgefäß vorgelegt und unter Rühren auf 90 °C erwärmt. Bei dieser Temperatur werden innerhalb 1 h 142,8 g Chloressigsäure zugegeben und danach noch weitere 5 h bei 95 °C nachgerührt. Die Analyse des Produkts erfolgt mittels HPLC.

Beispiel 6

410,3 g Amin aus Beispiel C) und 1 000 g Wasser werden in einem 2-Liter-Reaktionsgefäß vorgelegt und unter Rühren auf 90 °C erwärmt. Bei dieser Temperatur werden innerhalb 1 h 177,2 g Chloressigsäure zugegeben und danach noch weitere 5 h bei 95 °C nachgerührt. Die Analyse des Produkts erfolgt mittels HPLC.

Die Beispiele 1 bis 6 sind in Tabelle 1 zusammengefaßt :

Die mikrobizide Wirkung der erfindungsgemäßen Verbindungen und Gemische zeigt Tabelle 2 (μg Wirkstoff/ml Wasser ; Kontaktzeit 24 und 48 h ; Raumtemperatur). Die angegebenen Zahlen bedeuten die minimalen Hemmkonzentrationen, um die vorgelegte Keimzahl von $10^6$ Keimen pro ml abzutöten.

(Siehe Tabellen Seite 8 ff.)

Tabelle 1

| Beispiel | Zusammensetzung des Amingemisches VI' in mol | | Säure oder Säurederivat (mol) | Amphotere Verbindung I' (mol) | Amphotere Verbindung II' (mol) | Amphotere Verbindung III' (mol) |
|---|---|---|---|---|---|---|
| | Triamin | Diamin | | | | |
| 1 | 0,96 | 0,04 | 1,05 | 0,91 | 0,04 | 0,04 |
| 2 | 0,96 | 0,04 | 1,03 | 0,92 | 0,04 | 0,03 |
| 3 | 0,82 | 0,18 | 1,05 | 0,77 | 0,17 | 0,04 |
| 4 | 0,81 | 0,19 | 1,25 | 0,63 | 0,18 | 0,17 |
| 5 | 0,65 | 0,35 | 1,05 | 0,63 | 0,34 | 0,02 |
| 6 | 0,65 | 0,35 | 1,25 | 0,48 | 0,33 | 0,18 |

Die Analyse der Produkte erfolgt mittels High Performance Liquid Chromatography (HPLC) am Umkehrphasensystem (RP-18) unter Ionenpaarbedingungen in methanolisch-wäßrigem System an einem Hewlett-Packard 1082B Liquid Chromatograph.

Tabelle 2

| Mikroorganismen | Produkt aus Beispiel 1 | | Produkt aus Beispiel 3 | | Produkt aus Beispiel 5 | |
|---|---|---|---|---|---|---|
| | 24 h | 48 h | 24 h | 48 h | 24 h | 48 h |
| Staphylococcus aureus | 125 | 62,5 | 250 | 62,5 | 125 | 62,5 |
| Escherichia coli | 62,5 | 7,8 | 62,5 | 15,6 | 125 | 15,6 |
| Pseudomonas aeruginosa | 31,2 | 15,6 | 31,2 | 7,6 | 62,5 | 15,6 |
| Proteus mirabilis | 125 | 31,2 | 31,2 | 15,6 | 125 | 31,2 |
| Candida albicans | 125 | 15,6 | 31,2 | 15,6 | 31,2 | 15,6 |
| Desulfovibrio desulfuricans 85 | 31,2 | 7,8 | 31,2 | 7,8 | 31,2 | 7,8 |
| Desulfovibrio desulfuricans 39 | 31,2 | 7,8 | 15,6 | 15,6 | 31,2 | 7,8 |
| Algizide Wirkung | 62,5 | | 31,2 | | 62,5 | |

**Patentansprüche**

1. Amphotere Verbindungen der Formel

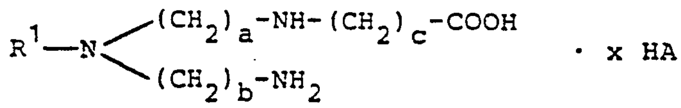

(I)

worin

$R^1$ ein Alkyl- oder Alkenylrest mit 8 bis 22 C-Atomen ist ;
A das Anion einer Mineral- oder Carbonsäure ist ;
a und b — unabhängig voneinander — Werte von 2 oder 3 ;
c Werte von 1 oder 2 annehmen kann ; und
x 0 ist oder ganzzahlige Werte von 1 bis 3 annehmen kann.

2. Amphotere Verbindungen der Formel I gemäß Anspruch 1, dadurch gekennzeichnet, daß
R einen Alkylrest mit 8 bis 14 C-Atomen bedeutet und
a und b den Wert 3 haben.

3. Desinfektionsreiniger-Zusammensetzungen, dadurch gekennzeichnet, daß sie
a) 80 bis 30 Mol-% einer amphoteren Verbindung der Formel I gemäß Anspruch 1, und
b) 20 bis 70 Mol-% einer amphoteren Verbindung der Formel

$$R^2{-}NH{-}(CH_2)_d{-}NH{-}(CH_2)_e{-}COOH \cdot y\ HA \quad (II),$$

worin

$R^2$ eine der in Formel I für $R^1$ genannten Bedeutungen hat ;
A das Anion einer Mineral- oder Carbonsäure ist,
d = 3 ist ; e Werte von 1 oder 2 annehmen kann und y 0 ist oder Werte von 1 oder 2 annehmen kann,
enthalten, wobei 0 bis 50 % der Molmenge von Komponente a) durch
c) eine amphotere Verbindung der Formel

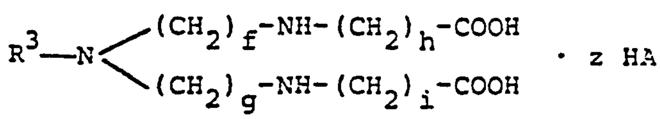

(III)

worin

$R^3$ eine der in Formel I für $R^1$ genannten Bedeutungen hat ;
A das Anion einer Mineral- oder Carbonsäure ist ;
f und g — unabhängig voneinander — Werte von 2 oder 3 haben ;
h und i — unabhängig von einander — Werte von 1 oder 2 haben ; und
z 0 ist oder ganzzahlige Werte von 1 bis 3 annehmen kann,
ersetzt sein kann.

4. Verfahren zur Herstellung von amphoteren Verbindungen der Formel I nach Anspruch 1, bei dem zunächst ein primäres Amin der Formel $R^1NH_2$ (IV), worin $R^1$ die in Formel 1 genannte Bedeutung hat, mit 2 mol mindestens eines reaktionsfähigen Nitrils mit 2 bis 3 C-Atomen zu einer Verbindung der Formel

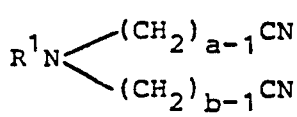

(V)

umgesetzt wird und diese in Gegenwart von Wasserstoff zu einer Verbindung der Formel

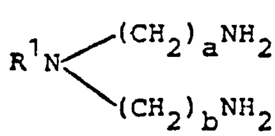

(VI)

reduziert wird, dadurch gekennzeichnet, daß diese Verbindung der Formel VI in wäßriger Lösung mit mindestens einer ω-Halogencarbonsäure der Formel $X(CH_2)_cCOOH$ (VII), worin X = Halogen ist und c die in Formel I genannte Bedeutung hat, oder mit Acrylsäure oder mit einem Alkali- oder Erdalkalisalz, einem Ester oder dem entsprechenden Nitril einer solchen Carbonsäure umgesetzt und gegebenenfalls zur Carbonsäure hydrolysiert und gegebenenfalls mit einer Mineral- oder Carbonsäure oder mit Alkali neutralisiert wird.

5. Verfahren zur Herstellung eines Gemisches aus
a') einer amphoteren Verbindung der Formel

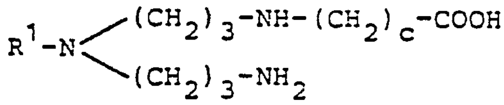 $\cdot$ x HA (I')

worin

$R^1$ ein Alkyl- oder Alkenylrest mit 8 bis 22 C-Atomen ist ;
A das Anion einer Mineral- oder Carbonsäure ist ;
c die Werte 1 und 2 ; und
x ganzzahlige Werte von 1 bis 3

annehmen kann,

b') einer amphoteren Verbindung der Formel

$$R^2—NH—(CH_2)_3—NH—(CH_2)_e—COOH \cdot y\ HA \qquad (II'),$$

worin

$R^2$ gleich $R^1$ ist ;
A das Anion einer Mineral- oder Carbonsäure ist ;
e Werte von 1 oder 2 ; und
y Werte von 1 oder 2 annehmen kann,

dadurch gekennzeichnet, daß ein primäres Amin der Formel IV, worin $R^1$ die in Formel I genannte Bedeutung hat, mit weniger als 2 mol, aber mehr als 1 mol eines reaktionsfähigen Nitrils mit 3 C-Atomen umgesetzt, in Gegenwart von Wasserstoff zu einem Gemisch VI' der Verbindungen der Formeln

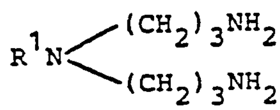

und

$$R^1N—(CH_2)_3—NH_2$$

reduziert wird und dieses Gemisch in wäßriger Lösung sodann mit einem Mol-Äquivalent mindestens einer ω-Halogencarbonsäure der Formel $X(CH_2)_cCOOH$ (VII), worin X = Halogen ist und c die in Formel I genannte Bedeutung hat ; oder mit Acrylsäure oder mit einem Alkali- oder Erdalkalisalz, einem Ester oder dem entsprechenden Nitril einer solchen Carbonsäure pro Mol-Äquivalent an vorhandenem tertiärem Amin-Stickstoff und einem weiteren Mol-Äquivalent pro Mol-Äquivalent an vorhandenem sekundärem Amin-Stickstoff umgesetzt und gegebenenfalls zur Carbonsäure hydrolysiert und gegebenenfalls mit einer Mineral- oder Carbonsäure oder mit Alkali neutralisiert wird.

6. Verfahren gemäß Anspruch 5 zur Herstellung eines Gemisches aus den Komponenten a'), b') und einer weiteren amphoteren Verbindung der Formel

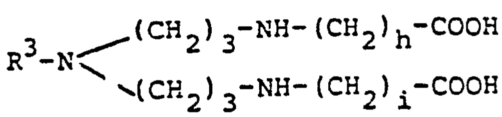 $\cdot$ z HA (III')

worin

$R^3$ gleich $R^1$ ist ;
A das Anion einer Mineral- oder Carbonsäure ist ;
h und i — unabhängig voneinander — die Werte 1 oder 2 ; und
z ganzzahlige Werte von 1 bis 3 annehmen kann,

dadurch gekennzeichnet, daß bei der Umsetzung mit ω-Halogencarbonsäuren, Acrylsäuren oder deren Derivaten pro Mol-Äquivalent an vorhandenem sekundärem Amin-Stickstoff 1 Mol-Äquivalent, pro Mol-Äquivalent an vorhandenem tertiärem Amin-Stickstoff aber mehr als 1 Mol-Äquivalent, höchstens aber 1,5 Mol-Äquivalent an der genannten ω-Halogencarbonsäure, Acrylsäure oder deren Derivaten eingesetzt wird.

7. Verwendung der amphoteren Verbindungen der Formel I gemäß Anspruch 1 als Desinfektionsmittel für die menschliche Haut und für Gegenstände mit harten Oberflächen.

**Claims**

1. Amphoteric compounds of the formula

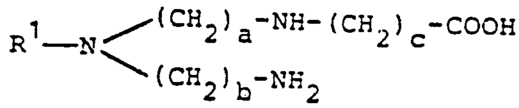 · x HA (I)

in which

$R^1$ is an alkyl or alkenyl radical having 8 to 22 carbon atoms ;
A is the anion of a mineral or carboxylic acid ;
a and b, independently of one another, can assume values of 2 or 3 ;
c can assume values of 1 or 2 ; and
x is 0 or can assume integral values from 1 to 3.

2. Amphoteric compounds of the formula I as claimed in claim 1, characterised in that R denotes an alkyl radical having 8 to 14 carbon atoms, and a and b have the value 3.

3. Disinfectant cleaning compositions characterised in that they contain
a) 80 to 30 mol-% of an amphoteric compound of the formula I as claimed in claim 1, and
b) 20 to 70 mol-% of an amphoteric compound of the formula

$$R^2—NH—(CH_2)_d—NH—(CH_2)_e—COOH \cdot y\ HA \quad (II)$$

in which

$R^2$ has one of the meanings mentioned for $R^1$ in formula I ;
A is the anion of a mineral or carboxylic acid,
d is 3 ; e can assume values of 1 or 2, and y is 0 or can assume values of 1 or 2,

from 0 to 50 % of the molar quantity of component a) being replaced by
c) an amphoteric compound of the formula

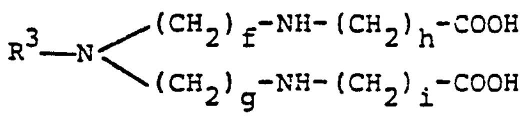 · z HA (III)

in which

$R^3$ has one of the meanings mentioned for $R^1$ in formula I ;
A is the anion of a mineral or carboxylic acid ;
f and g, independently of one another, have values of 2 or 3 ;
h and i, independently of one another, have values of 1 or 2 ; and
z is 0 or can assume integral values from 1 to 3.

4. A process for the preparation of amphoteric compounds of the formula I as defined in claim 1, in which first a primary amine of the formula $R^1NH_2$ (IV), in which $R^1$ has the meaning defined in formula I, is reacted with 2 moles of at least one reactive nitrile having 2 or 3 carbon atoms to give a compound of the formula

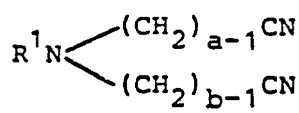 (V)

and the latter is reduced in the presence of hydrogen to give a compound of the formula

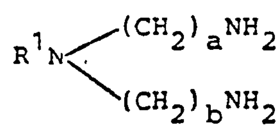 (VI)

which is characterised by reacting this compound of the formula VI, in aqueous solution, with at least one ω-halogenocarboxylic acid of the formula $X(CH_2)_cCOOH$ (VII), in which X is halogen and c has the meaning mentioned in formula I, or with acrylic acid, or with an alkali metal or alkaline earth metal salt, an ester or the corresponding nitrile of a carboxylic acid of this type, and, where appropriate, hydrolyzing to give the carboxylic acid and, where appropriate, neutralizing with a mineral or carboxylic acid or with alkali.

5. A process for the preparation of a mixture of
a') an amphoteric compound of the formula

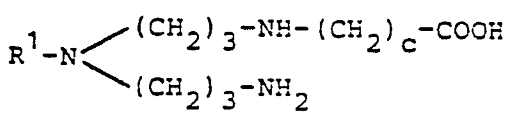 · x HA (I')

in which
$R^1$ is an alkyl or alkenyl radical having 8 to 22 carbon atoms ;
A is the anion of a mineral or carboxylic acid ;
c can assume the values 1 and 2 ; and
x can assume integral values from 1 to 3,
b') an amphoteric compound of the formula

$$R^2—NH—(CH_2)_3—NH—(CH_2)_e—COOH \cdot y\ HA \qquad (II')$$

in which
$R^2$ is identical to $R^1$ ;
A is the anion of a mineral or carboxylic acid ;
e can assume values of 1 or 2 ; and
y can assume values of 1 or 2,
which is characterised by reacting a primary amine of the formula IV, in which $R^1$ has the meaning mentioned in formula I, with less than 2 moles but more than 1 mole of a reactive nitrile having 3 carbon atoms, reducing in the presence of hydrogen to give a mixture VI' of the compounds of the formulae

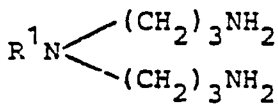

and

$$R^1N—(CH_2)_3—NH_2$$

and then reacting this mixture, in aqueous solution, with 1 mole-equivalent of at least one ω-halogenocarboxylic acid of the formula $X(CH_2)_cCOOH$ (VII), in which X is halogen and c has the meaning mentioned in formula I, or with acrylic acid or with an alkali metal or alkaline earth metal salt, an ester or the corresponding nitrile of a carboxylic acid of this type, per mole-equivalent of tertiary amine nitrogen present, and with a further mole-equivalent per mole-equivalent of secondary amine nitrogen present, and, where appropriate, hydroxyzing to give the carboxylic acid and, where appropriate, neutralizing with a mineral or carboxylic acid or with alkali.

6. The process as claimed in claim 5 for the preparation of a mixture of the components a'), b'), and another amphoteric compound of the formula

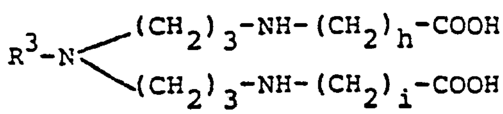 · z HA (III')

in which
$R^3$ is identical to $R^1$ ;
A is the anion of the mineral or carboxylic acid ;
h and i, independently of one another, can assume the values 1 or 2 ; and
z can assume integral values from 1 to 3,
which is characterized by using, in the reaction with ω-halogenocarboxylic acids, acrylic acids or their derivatives, 1 mole-equivalent per mole-equivalent of secondary amine nitrogen present, but using more than 1 mole-equivalent, but not more than 1.5 mole-equivalent, of the above-mentioned ω-halogenocarboxylic acid, acrylic acid or its derivatives per mole-equivalent of tertiary amine nitrogen present.

7. The use of the amphoteric compounds of the formula I, as claimed in claim 1, as disinfectants for human skin and for articles having hard surfaces.

**Revendications**

1. Composés amphotères de formule :

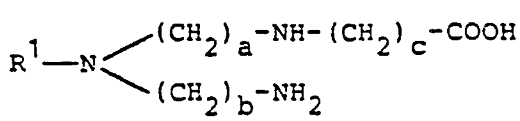 · x HA (I)

dans laquelle :
$R^1$ désigne un alkyle ou un alcényle ayant de 8 à 22 atomes de carbone ;
A l'anion d'un acide minéral ou carboxylique ;

a et b sont chacun, indépendamment l'un de l'autre, le nombre 2 ou 3 ;
c peut être le nombre 1 ou 2 ; et
x peut être le nombre 0, 1, 2 ou 3.

2. Composés amphotères de formule I selon la revendication 1 dans lesquels
R est un alkyle ayant de 8 à 14 atomes de carbone et
a et b sont chacun le nombre 3.

3. Produits de nettoyage désinfectants, caractérisés en ce qu'ils contiennent :
a) de 80 à 30 mol% d'un composé amphotère de formule I selon la revendication 1, et
b) de 20 à 70 mol% d'un composé amphotère de formule

$$R^2—NH—(CH_2)_d—NH—(CH_2)_e—COOH \cdot y\ A \quad (II)$$

dans laquelle :
$R^2$ a l'une des significations données pour $R^1$ à propos de la formule I,
A désigne l'anion d'un acide minéral ou d'un acide carboxylique,
d est le nombre 3, e le nombre 1 ou 2 et y le nombre 0, 1 ou 2,
de 0 à 50 % en moles du composant a) pouvant être remplacés par :
c) un composé amphotère de formule :

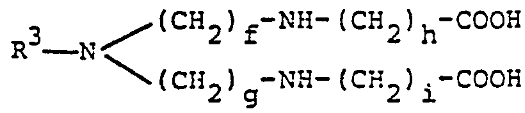 · z HA (III)

dans laquelle :
$R^3$ a l'une des significations indiquées pour $R^1$ à propos de la formule I,
A désigne l'anion d'un acide minéral ou d'un acide carboxylique,
f et g sont chacun, indépendamment l'un de l'autre, le nombre 2 ou 3,
h et i sont chacun, indépendamment l'un de l'autre, le nombre 1 ou 2, et
z est le nombre 0, 1, 2 ou 3.

4. Procédé de préparation de composés amphotères de formule I selon la revendication 1 dans lequel on fait d'abord réagir une amine primaire de formule $R^1NH_2$ (IV) avec 2 mol d'un ou de plusieurs nitriles réactifs ayant 2 ou 3 atomes de carbone pour former un composé de formule :

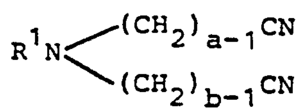 (V)

composé que l'on réduit avec de l'hydrogène en un composé de formule :

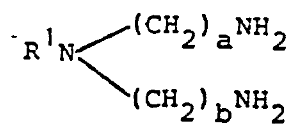 (VI)

procédé caractérisé en ce que l'on fait réagir ce composé de formule VI en solution aqueuse avec un ou plusieurs acides ω-halogénocarboxyliques de formule $X(CH_2)_cCOOH$ (VII), X désignant un halogène et c ayant la valeur indiquée à la revendication 1 pour la formule I, ou bien avec l'acide acrylique ou un sel de métal alcalin ou alcalino-terreux, un ester ou le nitrile correspondant d'un tel acide carboxylique, et le cas échéant on effectue une hydrolyse pour obtenir l'acide carboxylique et on neutralise éventuellement avec un acide minéral ou un acide carboxylique ou avec un alcali.

5. Procédé de préparation d'un mélange de
a') un composé amphotère de formule :

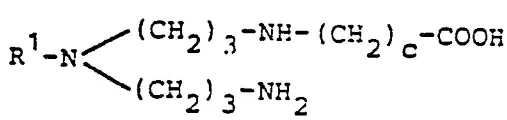 · x HA (I')

dans laquelle :
$R^1$ est un alkyle ou un alcényle en $C_8$ à $C_{22}$ ;
A est l'anion d'un acide minéral ou d'un acide carboxylique ;
c est le nombre 1 ou 2 ; et
x peut être le nombre 1, 2 ou 3, et
b') un composé amphotère de formule :

$R^2$—NH—$(CH_2)_3$—NH—$(CH_2)_e$—COOH · y HA (II'),

dans laquelle

$R^2$ est identique à $R^1$ ci-dessus ;

A est l'anion d'un acide minéral ou d'un acide carboxylique :

e est le nombre 1 ou 2 ; et

y le nombre 1 ou 2,

procédé caractérisé en ce que l'on fait réagir une amine primaire de formule IV, $R^1$ ayant la signification donnée pour la formule I, avec moins de 2 mol mais plus de 1 mol d'un nitrile réactif ayant 3 atomes de carbone, on effectue ensuite une réduction par l'hydrogène pour obtenir un mélange VI' des composés de formules :

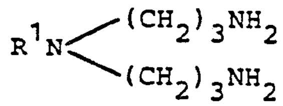

et

$R^1N$—$(CH_2)_3$—$NH_2$

puis on fait réagir ce mélange en solution aqueuse avec un équivalent molaire d'un ou de plusieurs acides ω-halogénocarboxyliques de formule $X(CH_2)_cCOOH$ (VII), X désignant un halogène et c ayant la valeur donnée pour la formule I, ou bien avec l'acide acrylique ou avec un sel de métal alcalin ou alcalino-terreux, un ester ou le nitrile correspondant d'un tel acide carboxylique, par équivalent molaire d'azote aminé tertiaire, et un autre équivalent molaire par équivalent molaire d'azote aminé secondaire, et le cas échéant on hydrolyse en acide carboxylique et on neutralise éventuellement avec un acide minéral ou un acide carboxylique ou avec un alcali.

6. Procédé selon la revendication 5 pour la préparation d'un mélange des composants a') et b') et d'un autre composé amphotère de formule :

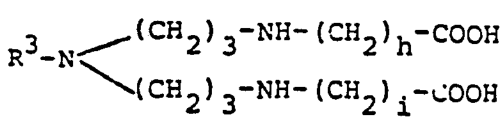 · z HA (III')

dans laquelle :

$R^3$ est identique à $R^1$ ;

A est l'anion d'un acide minéral ou d'un acide carboxylique ;

h et i sont chacun, indépendamment l'un de l'autre, le nombre 1 ou 2 ; et

z est le nombre 1, 2 ou 3,

procédé caractérisé en ce que dans la réaction avec l'acide ω-halogénocarboxylique, l'acide acrylique ou leurs dérivés on met en réaction, par équivalent molaire d'azote aminé secondaire, un équivalent molaire, et par équivalent molaire d'azote aminée tertiaire plus d'un équivalent molaire mais pas plus de 1,5 équivalent molaire, de l'acide ω-halogénocarboxylique, d'acide acrylique ou de leurs dérivés.

7. Utilisation des composés amphotères de formule I selon la revendication 1 comme produits de désinfection de la peau humaine et pour des articles à surfaces dures.